# EUROPEAN PATENT APPLICATION

(11) **EP 4 085 922 A1**
(43) Date of publication of application: **09.11.2022**
(21) Application number: 21305594.0
(22) Date of filing: 07.05.2021
(51) Int. Cl.: A61K 38/17, A61P 25/02

(54) **HIP/PAP PROTEIN OR A DERIVATIVE THEREOF FOR TREATING PERIPHERAL NEUROPATHY**

(71) Applicant: THE HEALTHY AGING COMPANY, 75001 Paris (FR)
(72) Inventor: BRECHOT, Christian, 75006 Paris (FR); AMOUYAL, Gilles, 75016 Paris (FR); AMOUYAL, Paul, 75016 Paris (FR); SANTORO, Lyse, 75016 Paris (FR); ROTH, Fanny, 94800 Villejuif (FR); JAMOT, Laure, 75013 Paris (FR)
(74) Representative: Nony

(57) **Abstract**

The present invention relates to the use of the HIP/PAP protein, or a derivative thereof, for treating or preventing peripheral neuropathy in a individual, in particular diabetic peripheral neuropathy in a diabetic or prediabetic individual. It also relates to the use of the HIP/PAP protein, or a derivative thereof, for preventing a complication of peripheral neuropathy in a individual, and in particular for preventing a diabetic foot wound, in particular a diabetic foot ulcer, and its consequences.

## Description

### Field of the invention

The present invention relates to the use of the HIP/PAP protein, or a derivative thereof, in the treatment and prevention of peripheral neuropathy in an individual, in particular diabetic peripheral neuropathy in an individual, as well as the prevention of disorders directly caused by such peripheral neuropathy, such as diabetic foot wound, in particular diabetic foot ulcers, and its consequences.

### Prior art

Peripheral neuropathies are conditions involving damage to the peripheral nervous system, the vast communication network that sends signals between the central nervous system (the brain and spinal cord) and all other parts of the body.

Symptomatic acquired peripheral neuropathies can originate from several different causes: a physical injury (trauma) such as car accidents, falls, sports and medical procedures or arthritis; diabetes; vascular and blood problems; systemic autoimmune diseases; autoimmune diseases that attack nerves only; hormonal imbalances; nutritional or vitamin imbalances; alcoholism; exposure to toxins (some medications, such as chemotherapy drugs); some cancers or benign tumors; or infections.

Diabetic peripheral neuropathy is the most common complication of diabetes and affects up to 50% of diabetic patients. It occurs in both type 1 and type 2 diabetes patients with higher prevalence in type 2 diabetes patients

Diabetic peripheral neuropathy is characterized by progressive distal to proximal degeneration of peripheral nerve axons, pain and loss of sensation as a consequence of nerve damage caused by chronically high blood sugar levels. It leads to numbness, loss of sensation and sometimes pain in the feet, legs or hands. As a consequence, diabetic peripheral neuropathy deteriorates the quality of life of diabetes patients and is the greatest source of diabetic patient mortality.

Moreover, diabetic peripheral neuropathy can cause a range of complications including chronic pain; foot deformation; foot wounds, and in particular foot ulcers; foot infections; and amputations. Indeed, damage to the innervations of the intrinsic foot muscles leads to an imbalance between flexion and extension of the affected foot. This produces anatomic foot deformities that create abnormal bony prominences and pressure points, which gradually cause skin breakdown and ulceration. Moreover, autonomic neuropathy leads to diminished sweating, which leads to the overlying skin becoming dry and increasingly susceptible to fissures and a subsequent development of infection. The loss of sensation as a part of peripheral neuropathy exacerbates the development of ulcerations. As trauma occurs at the affected site, patients are unable to detect the insult to their lower extremities. As a result, many wounds go unnoticed and progressively worsen as the affected area is continuously subjected to repetitive pressure and shear forces from ambulation and weight bearing. Furthermore, the autonomic denervation leads to bone demineralization via the impairment of the vascular smooth muscle, which leads to an increase in blood flow to the bone with consequential osteolysis. (Sharad P. Pendsey. Int J Diabetes Dev Ctries. 2010 Apr-Jun; 30(2): 75-79)

The estimated global prevalence of diabetic foot ulcers is 6%, with major risk factors including high size, older age, lower body mass index, longer duration of diabetes, hypertension, diabetic retinopathy, and smoking (Global epidemiology of diabetic foot ulceration: a systematic review and meta-analysis. Zhang P. et al. Ann Med. 2017 Mar;49(2):106-116*.).* Approximately 25% of people with diabetes will develop a foot ulcer during their lifetime, which can progress to infection and limb amputation in severe cases. Of all amputations in diabetic patients, 85% are preceded by a foot ulceration which subsequently deteriorates to a severe gangrene or infection. For example, in the United States where the highest prevalence of diabetic foot ulceration was reported, diabetes accounts for 83% of all major amputations. The total medical cost for treating diabetic foot diseases in America ranges from $9 to $13 billion and is an additional cost associated with diabetes (Rice JB et al. Burden of diabetic foot ulcers for medicare and private insurers. Diabetes Care. 2014;37:651-8).

As the correlation between hyperglycemia and the progression of neuropathy is well established and has been revealed by many studies delineating the high prevalence rates of diabetic peripheral neuropathy in diabetes patients who have not kept their blood sugar under control, controlling the blood sugar has been recognized as a primary method of prevention/treatment of diabetic peripheral neuropathy (N Engl J Med 1993;329:977-86). This method has however demonstrated its limits and most diabetic patients are not able to keep their blood sugar under control.

Other solutions have been studied under the form of therapeutic agents. For example, alpha lipoic acid, aldose reductase inhibitors, and gamma linoleic acid. α-Lipoic acid (thioctic acid), an antioxidant, have be identified as reducing the hyperglycemia-induced oxidative stress which leads to the death of nerve cells, thereby blocking the etiology of diabetic neuropathy. Aldose reductase inhibitors can prevent glucose from entering the polyol pathway in nerve cells, which can suppress the accumulation of sorbitol, leading to nerve cell damage, to block the progression of diabetic neuropathy. However, the development of most of these actives was stopped halfway due to side effects and insufficient efficacy. Y-Linolenic acid, which is both a component of nerve membrane phospholipids and a component of prostaglandin E and plays an important role in the homeostasis of blood flow in nerve cells, is found to ameliorate some symptoms of diabetic neuropathy.

With the number of cases and the prevalence of diabetes steadily increasing worldwide over the past few decades, and the estimated number of people suffering from diabetes being estimated by the World Health Organization at well above 420 million (with more than 1.6 million deaths directly attributed to diabetes each year), finding new solutions to prevent and treat diabetic peripheral neuropathy remains an important goal in terms of public health.

Therefore, there is an increasing need for novel actives able to prevent and/or treat peripheral neuropathy, and in particular diabetic peripheral neuropathy.

Moreover, there is an escalating need for the prevention of the complications of peripheral neuropathy, more particularly diabetic peripheral neuropathy, and in particular for the prevention of foot wounds, and in particular foot ulcers; foot infections; and amputations thereof.

### Summary of the invention

The applicant has demonstrated that, surprisingly, the HIP/PAP protein, or a derivative thereof, makes it possible to prevent and/or treat peripheral neuropathy in a patient, and in particular to prevent and/or treat diabetic peripheral neuropathy in diabetic or prediabetic patients. The HIP/PAP protein may therefore advantageously be used for:
- treating or preventing peripheral neuropathy in patients, and in particular diabetic peripheral neuropathy in diabetic or prediabetic patients; and
- preventing complications of peripheral neuropathy, and in particular of diabetic peripheral neuropathy, in particular preventing diabetic foot wounds, more particularly diabetic foot ulceration.

The present invention accordingly relates to the following items:
**Item 1:** A HIP/PAP protein, or a derivative thereof, for its use in treating and/or preventing peripheral neuropathy in an individual.
**Item 2:** The HIP/PAP protein, or a derivative thereof, for use according to item 1 in treating and/or preventing diabetic peripherical neuropathy, in particular in a diabetic or prediabetic individual, more particularly in a diabetic individual.
**Item 3:** A HIP/PAP protein, or a derivative thereof, for its use in preventing a complication of peripheral neuropathy in an individual, and in particular preventing a complication of peripheral neuropathy in a diabetic or prediabetic individual, more particularly preventing a complication of diabetic peripheral neuropathy in a diabetic or prediabetic individual, in particular in a diabetic individual.
**Item 4:** The HIP/PAP protein, or a derivative thereof, for use according to item 3, wherein the complication of diabetic peripheral neuropathy is selected from the group consisting of foot deformation; diabetic foot wound, and in particular diabetic foot ulcer; foot infection; and amputations.
**Item 5:** The HIP/PAP protein, or a derivative thereof, for use according to any one of items 1 to 4, characterized in that the individual is a mammal, in particular a human being.
**Item 6:** The HIP/PAP protein, or a derivative thereof, for use according to any one of items 1 to 5, characterized in that the diabetic individual has a type II diabetes.
**Item 7:** The HIP/PAP protein, or a derivative thereof, for use according to any one of items 1 to 6, characterized in that the HIP/PAP protein comprises an amino acid sequence selected from the group consisting of sequences set forth as SEQ ID NO: 1, SEQ ID NO: 2, SEQ ID NO: 3 and SEQ ID NO: 4.
**Item 8:** The HIP/PAP protein, or a derivative thereof, for use according to any one of items 1 to 7, wherein said derivative comprises an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4.
**Item 9:** The HIP/PAP protein, or a derivative thereof, for use according to any one of items 1 to 8, wherein said HIP/PAP protein, or a derivative thereof, is administered to the said individual in combination with at least one agent known for being useful in the prevention and/or treatment of peripheral neuropathy, in particular selected from the group consisting of steroids, anti-inflammatory drugs, immunosuppresants and Vitamin D.
**Item 10:** The HIP/PAP protein, or a derivative thereof, for use according to any one of items 1 to 9, wherein said HIP/PAP protein, or a derivative thereof, is administered to the said individual in combination with at least one anti-diabetic agent, in particular selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.
**Item 11:** The HIP/PAP protein, or a derivative thereof, for use according to any one of items 1 to 10, characterized in that the HIP/PAP protein, or a derivative thereof, is in a composition comprising a physiologically acceptable medium.
**Item 12:** The HIP/PAP protein, or a derivative thereof, for use according to item 11, wherein the composition is for oral, sublingual, subcutaneous, intramuscular, intravenous, topical, local, intratracheal, intranasal or rectal administration.
**Item 13:** The HIP/PAP protein, or a derivative thereof, for use according to item 11 or 12, wherein the composition is for oral, subcutaneous, intravenous, topical or local administration.
**Item 14:** The HIP/PAP protein, or a derivative thereof, for use according to any one of items 11 to 13, wherein the composition further comprises at least one agent known for being useful in the prevention and/or treatment of peripheral neuropathy in particular selected from the group consisting of steroids, anti-inflammatory drugs, immunosuppresants and Vitamin D.
**Item 15:** The HIP/PAP protein, or a derivative thereof, for use according to any one of items 11 to 14, wherein the composition further comprises at least one anti-diabetic agent, in particular selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.

### BRIEF DESCRIPTION OF THE FIGURES

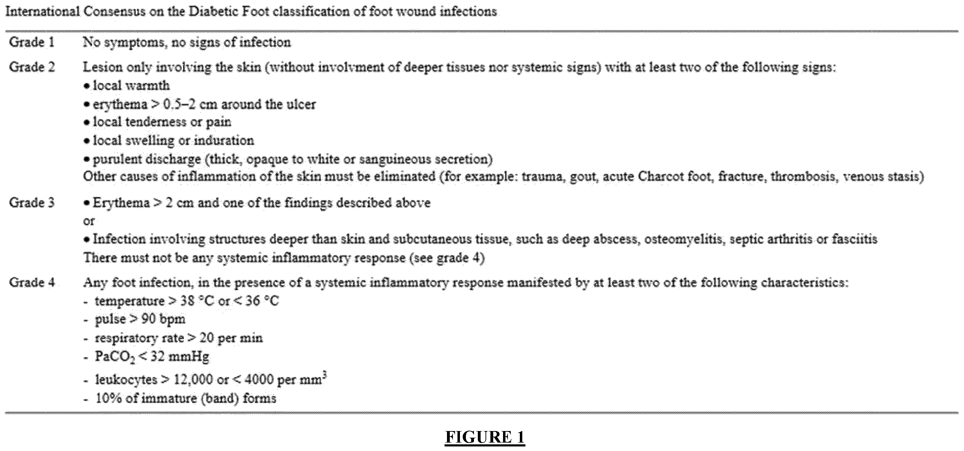

Figure 1 is a Table representing the severity of infection assessed according to the International Consensus on the Diabetic Foot classification system

### Detailed description of the invention

### Definitions

In the context of the present invention, the terms *"prevent", "prevention"* and *"preventing"* denote the reduction to a lesser degree of the risk or of the probability of occurrence of a given phenomenon, that is to say, in the present invention, the prevention of the onset of peripheral neuropathy and/or of complications thereof, in particular of complications selected from the group consisting of diabetic foot ulcer, foot infection and amputations. The terms *"prevent", "prevention"* and *"preventing"* also include preventing the aggravation, reducing the rate of progression or preventing reccurence of the said given phenomenon.

As used herein, the terms *"treating", "treatment"* or *"treat"* include alleviation of the symptoms associated with a specific disorder or condition and/or elimination of said symptoms, that is to say, in the present invention, the treatment of diabetic peripheral neuropathy.

A "diabetic" individual refers to the generally accepted definition of this term, i.e. to an individual suffering from a progressive disease of carbohydrate metabolism involving inadequate production or utilization of insulin, frequently characterized by hyperglycemia and glycosuria.

By "prediabetic" individual, it is referred to an individual that does not have the characteristics, symptoms and the like typically observed in diabetes, but does have characteristics, symptoms and the like that, if left untreated, can progress to diabetes. The presence of these conditions can be determined using, for example, either the fasting plasma glucose (FPG) test or the oral glucose tolerance test (OGTT). In the FPG test, a subject's blood glucose is measured after the conclusion of the fasting; generally, the subject fasts overnight and the blood glucose is measured in the morning before the subject eats. A healthy subject would generally have a FPG concentration between about 90 mg/dL and about 100 mg/dL, a subject with "pre-diabetes" would generally have a FPG concentration between about 100 mg/dL and about 125 mg/dL, and a subject with "diabetes" would generally have a FPG level above about 126 mg/dL. In the OGTT, a subject's blood glucose is measured after fasting and again two hours after drinking a glucose-rich beverage. Two hours after consumption of the glucose-rich beverage, a healthy subject generally has a blood glucose concentration below about 140 mg/dL, a pre-diabetic subject generally has a blood glucose concentration about 140 mg/dL to about 199 mg/dL, and a diabetic subject generally has a blood glucose concentration about 200 mg/dL or above.

*"Complications of peripheral neuropathy"* according to the present invention are medical problems caused by peripheral neuropathy. Examples of such complications of peripheral neuropathy are skin and limb trauma or burns, as the individual can have an altered sensitivity to temperatures or pain, and in particular diabetic skin and limb trauma or burns; foot deformity; heart and blood circulation problems; foot wounds, in particular diabetic foot wounds, and more particularly diabetic foot ulcers; gangrene; and foot infections and amputations of a limb, in particular of a lower limb.

According to the invention, *"foot wounds"* are wounds on the skin of a foot either due to a trauma (i.e. an external force, consequence for example of a accident (a fall for example) due to damages to the motor and/or sensory nerves), or to a gradual disturbance of tissues by an underlying (and thus internal) etiology/pathology, such as in the case of a diabetic foot ulcer as explained above.

*"Diabetic foot wounds"* according to the present invention is a feet wound as defined above occurring in a diabetic individual and being the consequence of his diabetic peripheric neuropathy as explained above. A diabetic foot wound according to the invention is in particular a diabetic foot ulcer.

The development of a diabetic foot ulcer is usually in 3 stages. The initial stage is the development of a callus, which results from diabetic peripheral neuropathy. The neuropathy causes physical deformity of the foot and sensory loss which leads to ongoing trauma. Drying of the skin because of neuropathy is also another contributing factor. Finally, frequent trauma of the callus results in subcutaneous hemorrhage and it erodes and becomes an ulcer.

*"Diabetic foot infection"* according to the present invention is an infection of the foot, i.e. invasion of the tissues with proliferation of microorganisms causing tissue damage with or without an associated inflammatory response by the diabetic individual.

The diagnosis of diabetic foot infection is clinical. The diagnosis ofinfection is based on the presence of at least two of the follo-wing signs: swelling, induration, erythema around the lesion, local tenderness or pain, local warmth or presence of pus. The severity of infection is assessed according to the International Consensus on the Diabetic Foot classification system (See Figure 1).

*"Amputation",* according to the present invention, is the removal by surgery of body part(s) of an diabetic individual, in particular lower limbs amputations.

Amputation surgery sometimes remains the only option in the case of severe, deep infection and/or wounds. The choice of the level of amputation depends on the vascular status, while taking every effort to preserve heel weight-bearing with the prosthesis. The most common amputations in people with diabetes are the toes, feet, and lower legs. In particular, major amputations are required in patients with overwhelming sepsis or deep compartment abscesses with extensive forefoot gangrene or impending toe loss. Aim of amputation is to preserve the extremity length, as longer the stump the better are the rehabilitation results.

The terms *"anti-diabetic agent"* designate a a substance that helps a person with diabetes control their level of glucose in the blood. Examples of anti-diabetic agents can for example be anti-diabetic agents selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.

Dipeptidyl peptidase 4 (DPP-4) inhibitors are a class of medicine that lower high blood glucose levels and may be used in the treatment of type 2 diabetes. Mention may be made of saxagliptin, sitagliptin, alogliptin and linagliptin.

Glucagon-like peptide-1 receptor agonists, also known as GLP-1 receptor agonists or incretin mimetics, are agonists of the GLP-1 receptor used for the treatment of type 2 diabetes. Mention may be made of exenatide, lixisenatide, liraglutide, albiglutide, dulaglutide, and semaglutide.

Sodium-glucose cotransporter 2 inhibitors are a class of medicine used to lower high blood glucose levels in people with type 2 diabetes. Mention may be made of ertugliflozin, canagliflozin, empagliflozin and dapagliflozin.

The term *"physiologically acceptable medium"* is intended to denote a medium which is compatible with the body of the individual to whom said composition must be administered. It is, for example, a non-toxic solvent such as water. In particular, said medium is compatible with oral, sublingual, subcutaneous, intramuscular, intravenous, topical, local, intratracheal, intranasal or rectal administration, and more particularly with oral, subcutaneous, intravenous, topical or local administration.

As used herein, the *"percentage of identity"* between two amino acid sequences is determined by comparing both optimally aligned sequences through a comparison window.

The portion of the amino acid sequence in the comparison window may thus include additions or deletions (for example "gaps") as compared to the reference sequence (which does not include these additions or these deletions) so as to obtain an optimal alignment between both sequences.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences may be determined using the Needleman and Wunsch algorithm for the alignment of two sequences. (Needleman, S. B. and Wunsch, C. D. (1970) J. Mol. Biol. 48, 443-453). Both amino acid sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences, EBLOSUM62 is used for the substitution matrix. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

As used herein, the term *"polypeptide"* refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms *"protein", "peptide"* and *"polypeptide"* can be used interchangeably. Polypeptides may optionally be modified (e.g., glycosylated, phosphorylated, acylated, farnesylated, prenylated, sulfonated, and the like) to add functionality.

### HIP/PAP protein and derivative thereof according to the invention

The HIP/PAP protein is known for its anti-apoptotic and mitogenic activity on hepatic cells (US 13/032,521, WO 2004/112824, Simon et al., FASEB J. 2003 Aug;17(11):1441-50).

It has also been shown that a 15-amino acid peptide, derived from the Reg IIIa family (HIP/PAP), the HIP (Human proIslet Peptide) peptide, has a regenerating activity on pancreatic islets and thus stimulates insulin production (US 2010/0093605).

The HIP/PAP protein according to the invention can comprise an amino acid sequence selected from the group consisting of sequences set forth as SEQ ID NO: 1, SEQ ID NO: 2, SEQ ID NO: 3 and SEQ ID NO: 4.

The amino acid sequence SEQ ID NO: 1 corresponds to the HIP/PAP protein of sequence SEQ ID NO: 4, from which the N-terminal 26-amino acid signal peptide of said protein has been deleted.

In a particular embodiment, the HIP/PAP protein according to the invention comprises, or consists of, the amino acid sequence set forth as SEQ ID NO: 4.

In a particular embodiment, the HIP/PAP protein according to the invention comprises, or consists of, the amino acid sequence set forth as SEQ ID NO: 1.

The amino acid sequence SEQ ID NO: 2 corresponds to the short form of the HIP/PAP protein and, compared with the amino acid sequence SEQ ID NO: 1, has had the 11-amino acid propeptide in the N-terminal position deleted.

In a particular embodiment, the HIP/PAP protein according to the invention comprises, or consists of, the amino acid sequence set forth as SEQ ID NO: 2.

The sequence SEQ ID NO: 3 corresponds to the sequence SEQ ID NO: 1, to which a methionine has been added in the N-terminal position. The HIP/PAP derivative of sequence SEQ ID NO: 3 is also called rcHIP/PAP or ALF5755. This derivative may in particular be produced recombinantly in E.Coli cells. The 12-amino acid N-terminal propeptide (propeptide of 11 amino acids plus the additional methionine) may be cleaved, in order to obtain the short form of the HIP/PAP protein (SEQ ID NO: 2).

In a particular embodiment, the HIP/PAP protein according to the invention comprises, or consists of, the amino acid sequence set forth as SEQ ID NO: 3.

According to the invention, the short or long forms of the HIP/PAP protein or of the derivatives thereof may be used indifferently.

A derivative of the HIP/PAP protein according to the invention designates a biologically active derived form of a HIP/PAP protein of any one of sequences SEQ ID No. 1 to 4. The terms "biologically active" mean that the derivative of the HIP/PAP protein have the same biological activity as the HIP/PAP protein of any one of sequences SEQ ID No. 1 to 4.

A derivative of the HIP/PAP protein according to the invention comprises, or consists in, an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4.

A biological activity of the same nature as the HIP/PAP protein according to the present invention is as described previously, i.e. the capacity to treat and/or prevent peripheral neuropathy in an individual, in particular a diabetic peripheral neuropathy, and to accordingly prevent a complication of peripheral neuropathy in an individual, in particular a complication as detailed elsewhere in the present text.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

In certain embodiments, the HIP/PAP protein, or a derivative thereof, may be associated or combined by noncovalent bonds with non-HIP/PAP portions. For example, the HIP/PAP protein or a derivative thereof could be associated with a liposome particle. Depending on the type of liposome, or on the production process, the HIP/PAP protein, or the derivative thereof, may be associated at the surface of the liposome or encapsulated inside said liposome.

The HIP/PAP protein, or a derivative thereof, may also be associated with non-HIP/PAP portions by covalent bonds. Such non-HIP/PAP portions may be selected from protein or non-protein compounds, for example polyethylene glycol, thus forming a pegylated HIP/PAP derivative.

A derivative of the HIP/PAP protein according to the invention also comprises the derivatives which become biologically active only when they are administered to the patient.

Finally, the derivatives of the HIP/PAP protein also comprise chimeric proteins or fusion proteins. Such proteins are fused with non-HIP/PAP polypeptides. The latter may be fused with the N- or C-terminal part. Typically, the HIP/PAP protein or a derivative thereof may be fused with the GST sequence at the level of their C-terminal part, in order to facilitate the purification of the recombinant proteins.

In certain embodiments of the invention, the HIP/PAP protein, or a derivative thereof, according to the invention is produced recombinantly in bacterial or animal cells, including insect and mammalian cells, according to techniques known to those skilled in the art.

In other embodiments, the HIP/PAP protein, or a derivative thereof, according to the invention may be isolated from a cell or from a tissue by known purification techniques.

The HIP/PAP protein and the derivatives thereof may also be produced by chemical synthesis.

In the text, the terms "HIP/PAP protein" cover the HIP/PAP protein in itself, and also the derivatives thereof as described above.

### Compositions

The invention also relates to the implementation of the HIP/PAP protein, or a derivative thereof, as defined previously in a composition comprising a physiologically acceptable medium.

The physiologically acceptable medium, previously defined in the present text, may be chosen, according to the pharmaceutical form and the mode of administration desired, from the usual excipients which are known to those skilled in the art (see Remington's Pharmaceutical Sciences, 16th edition, Osol, A ed., 1980).

By way of example, compositions according to the invention may comprise, according to the therapeutic indications and the HIP/PAP protein or the derivative thereof:
a) the HIP/PAP protein or a derivative thereof; and
b) a buffer capable of maintaining the pH in a maximum stability range, preferentially from 1 to 9, more particularly from 4 to 8 and even more particularly from 6 to 7.5; and/or
c) a detergent or a surfactant which stabilizes the protein or the polypeptide against the aggregation induced by stirring; and/or
d) an isotonic; and/or
e) a preservative, chosen for example from the group consisting of phenols, benzyl alcohols, benzothelium halides, and chlorides; and/or
f) water.

If the detergent or the surfactant used is nonionic, it may be chosen from polysorbates, PLURONIC TM, polyethylene glycol (PEG) or poloxamers.

An isotonic will make it possible to maintain the isotonicity of the composition and will typically include polyalcohols such as glycerol, erythritol, arabitol, xylitol, sorbitol or mannitol, used alone or in combination. Alternatively, sodium chloride and/or any other inorganic salt may be used as isotonic.

The buffer may be, for example, acetate, citrate, succinate, a phosphate buffer or any other inorganic buffer, depending on the desired pH.

The preservatives of phenol, benzyl alcohol, benzothelium halide and chloride type are known antimicrobial agents. Typical preservatives comprise octadecyldimethylbenzylammonium chloride, hexamethonium chloride, benzalkonium chloride, phenol, butyl or benzyl alcohols, alkyl parabens, such as methyl or propyl paraben, catechol, resorcinol, cyclohexanol, 3-pentanol and m-cresol.

Additional excipients may also comprise antioxidants, such as ascorbic acid and methionine, chelating agents, such as EDTA, sugars, such as sucrose, mannitol, trehalose or sorbitol, etc.

The HIP/PAP protein, or the derivative thereof, according to the invention may be in the form of a pharmaceutically acceptable salt. This is intended to mean salts prepared from pharmaceutically acceptable non-toxic acids or pharmaceutically acceptable non-toxic bases, including organic and inorganic salts and acids. By way of example, mention may be made of alkali metal salts (sodium and potassium salts), alkaline-earth metal salts (calcium and magnesium salts), ammonium salts, salts of organic bases (pyridine or triethylamine salts), salts of inorganic acids (hydrochloride, sulfate, nitrate) and salts of organic acids (acetate, oxalate, p-toluenesulfonate).

A composition implemented according to the invention may be for oral, sublingual, subcutaneous, intramuscular, intravenous, topical, local, intratracheal, intranasal or rectal administration, and in particular for oral, subcutaneous, intravenous, topical or local administration.

According to one preferred embodiment, the HIP/PAP protein is administered in an effective amount, i.e. the amount required to obtain the expected effects of the invention. Such an amount of HIP/PAP protein will be determined, generally empirically, according to the subject to be treated and to his pathological condition. The effective amount will also depend on the mode of administration envisioned. The adjustments required to determine the effective amount for obtaining the maximum therapeutic effect correspond to techniques that are routine for the clinician.

An effective amount of the HIP/PAP protein or of a derivative thereof may for exemple be between 0.1 µg/day/kg of body weight and 100 mg/day/kg of body weight of the individual to which it must be administered. Although in certain embodiments an effective amount of the HIP/PAP protein, or derivative thereof, may reach more than 10 mg/kg, an effective amount of the HIP/PAP protein, or derivative thereof, according to the present invention is generally less than 5 mg/kg of body weight, which includes amounts less than 4.5 mg/kg, 4 mg/kg, 3.5 mg/kg, 3 mg/kg, 2.5 mg/kg or 2000 µg/kg. More particularly, an effective amount of the HIP/PAP protein, or derivative thereof, according to the present invention comprises amounts of at least 1 µg/kg, 2 µg/kg, 3 µg/kg, 4 µg/kg, 5 µg/kg, 6 µg/kg, 7 µg/kg, 8 µg/kg, 9 µg/kg, 10 µg/kg, 15 µg/kg, 20 µg/kg, 25 µg/kg, 30 µg/kg, 40 µg/kg, 50 µg/kg, 60 µg/kg, 70 µg/kg, 80 µg/kg, 90 µg/kg, 100 µg/kg, 150 µg/kg, 200 µg/kg, 250 µg/kg, 300 µg/kg, 350 µg/kg, 400 µg/kg, 450 µg/kg, 500 µg/kg, 600 µg/kg, 700 µg/kg, 800 µg/kg, 900 µg/kg, 1 mg/kg, 2 mg/kg, 3 mg/kg, 4 mg/kg, 5 mg/kg or more with respect to body weight of the individual to which it is or must be administered.

According to particular embodiments, the HIP/PAP protein, or derivative thereof, is administered according to a dosage of between 10 and 5000 µg/kg, preferentially between 100 and 2000 µg/kg with respect to body weight.

In the compositions of the present invention for oral, sublingual, subcutaneous, intramuscular, intravenous, topical, local, intratracheal or intranasal or rectal administration, the active ingredient (the HIP/PAP protein or a derivative thereof) may be administered in unit administration form as a mixture with pharmaceutical excipients.

When the composition is for oral administration, said composition may be chosen from the group consisting of a food product, a beverage, a pharmaceutical product, a nutraceutical, a food additive, a food supplement and a milk product.

The preferred modes of administration are the oral, subcutaneous, intravenous, topical or local routes, and more particularly the subcutaneous, intravenous, topical or local routes.

The administration of a compound or composition of the invention may for example be performed through the use of a sheath, a patch, a pad, a compress, a bandage, a tape, a gauze-based dressing, a woven or non-woven sponge or a syringe.

The HIP/PAP protein, or a derivative thereof, may be sterilized prior to the *in vivo* administration. The sterilization may be obtained by filtration on sterile filtration membranes, before or after the lyophilization or the reconstitution. The systemically administered HIP/PAP protein, or derivative thereof, may advantageously be lyophilized or stored in solution. In lyophilized form, the HIP/PAP protein, or derivative thereof, may be generally formulated in combination with excipients which enable reconstitution with an appropriate diluent, at the time of use.

The HIP/PAP protein, or a derivative thereof, may be administered daily in one intake or in a fractionated manner (for example from 2 to 3 times per day) until the desired therapeutic effect is obtained. It may also be administered chronically.

The HIP/PAP protein, or a derivative thereof, may also be administered in the form of a course, for example courses ranging from 15 days to 3 months, optionally repeated from 1 to 6 times at determined doses and time intervals.

The HIP/PAP protein, or a derivative thereof, according to the invention may also be combined in the context of a polytherapy with other compounds for treating and/or preventing peripheral neuropathy, in particular diabetic peripheral neuropathy, or for preventing a complication of peripheral neuropathy in an individual, and in particular for preventing a complication of diabetic peripheral neuropathy in a diabetic indivisual.

Accordingly, a HIP/PAP protein, a derivative thereof, or a composition comprising it according to the invention may be administered to the previously defined individual, in particular a pre-diabetic or diabetic individual, and more particularly a diabetic individual, in combination with at least one agent known for being useful in the prevention and/or treatment of peripheral neuropathy, and in particular in the prevention and/or treatment of diabetic peripheral neuropathy.

Such agents are known by the man skilled in the art and can for example be selected from the group consisting of steroids, anti-inflammatory drugs, immunosuppresants and Vitamin D.

Moreover, as previously mentioned, a HIP/PAP protein, a derivative thereof, or a composition comprising it according to the invention may be administered to the previously defined individual, in particular a pre-diabetic or diabetic individual, and more particularly a diabetic individual, in combination with at least one anti-diabetic agent.

Said anti-diabetic agent may be selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.

The dipeptidyl peptidase 4 (DPP-4) inhibitor may be selected from the group consisting of saxagliptin, sitagliptin, alogliptin and linagliptin.

The glucagon-like peptide-1 receptor agonist may be selected from the group consisting of exenatide, lixisenatide, liraglutide, albiglutide, dulaglutide, and semaglutide.

The sodium-glucose cotransporter 2 inhibitor may be selected from the group consisting of ertugliflozin, canagliflozin, empagliflozin and dapagliflozin.

By administered in combination with, it means that the HIP/PAP protein, a derivative thereof, or a composition comprising it according to the invention can be administered simultaneously or sequentially compared to the other compounds, in particular compared to the at least one anti-diabetic agent. When they are in separate compositions, the composition comprising the HIP/PAP protein, a derivative thereof, according to the invention and the composition comprising the at least one anti-diabetic agent can be administered through the same route or through different routes.

The HIP/PAP protein, or a derivative thereof, according to the invention and the at least one anti-diabetic agent can be administered in the same composition or in separate compositions.

By simultaneously, it is understood that the compositions can be administered at the same moment or up to the same day or couple of days.

By sequentially, it is understood that the compositions can be administered with at least several days, for example at least two days of difference.

### Implementation of the HIP/PAP protein and/or of a derivative thereof

As previously mentioned, a HIP/PAP protein, of a derivative thereof, as well as a composition comprising it, is for use in:
- the treatment and/or prevention of a peripheral neuropathy, and in particular of a diabetic peripheral neuropathy; and/or
- the prevention of a complication of a peripheral neuropathy, in particular of a diabetic peripheral neuropathy;
in an individual, in particular in a diabetic or prediabetic individual, more particularly in a diabetic individual.

As previously indicated, said complication of a peripheral neuropathy, and in particular of a diabetic peripheral neuropathy, may be selected from the group consisting of skin and limb trauma or burns, and in particular diabetic skin and limb trauma or burns; foot deformity; heart and blood circulation problems; foot wounds, in particular diabetic foot wounds, and more particularly diabetic foot ulcers; gangrene; and foot infections and amputations of a limb, in particular of a lower limb.

A complication of diabetic peripheral neuropathy according to the present invention may in particular be selected from the group consisting of foot deformation, diabetic foot wound, and in particular diabetic foot ulcer, foot infection and amputations.

An individual, and in particular a diabetic individual, to which the HIP/PAP protein, a derivative thereof, or a composition according to the invention is administered can in particular be a mammal and in particular a human being.

The HIP/PAP protein, a derivative thereof, or a composition according to the invention can be administered in combination with standards of medical care for peripheral neuropathy and/or a complication of peripheral neuropathy, and more particularly for diabetic peripheral neuropathy and/or a complication of diabetic peripheral neuropathy.

The present invention also relates to a method for treating and/or preventing peripheral neuropathy in an individual, in particular for treating and/or preventing diabetic peripheral neuropathy in a diabetic individual, comprising administering to the said individual a HIP/PAP protein, a derivative thereof, or a composition comprising it.

The present invention moreover relates to the use of a HIP/PAP protein, a derivative thereof, or a composition comprising it, for treating and/or preventing peripheral neuropathy in an individual, and in particular for treating and/or preventing diabetic peripheral neuropathy in a diabetic individual.

The present invention further relates to the use of a HIP/PAP protein, a derivative thereof, or a composition comprising it, for the manufacture of a medicament for treating and/preventing peripheral neuropathy in an individual, and in particular for treating and/preventing diabetic peripheral neuropathy in a diabetic individual.

The present invention also relates to a method for preventing a complication of peripheral neuropathy in an individual, and in particular preventing a complication of diabetic peripheral neuropathy in a diabetic or prediabetic individual, comprising administering to the said individual a HIP/PAP protein, a derivative thereof, or a composition comprising it.

The present invention moreover relates to the use of a HIP/PAP protein, a derivative thereof, or a composition comprising it, for preventing a complication of peripheral neuropathy in an individual, and in particular preventing a complication of diabetic peripheral neuropathy in a diabetic or prediabetic individual.

The present invention further relates to the use of a HIP/PAP protein, a derivative thereof, or a composition comprising it, for the manufacture of a medicament for preventing a complication of peripheral neuropathy in an individual, and in particular preventing a complication of diabetic peripheral neuropathy in a diabetic or prediabetic individual.

The invention is described below in greater detail by means of the following examples which are given solely by way of illustration.

All the references to percentages are percentages by weight unless otherwise indicated.

### EXAMPLES

The effect of a 2-month systemic treatment with ALF-5755 (compound of sequence SEQ ID NO: 3) on peripheral neuropathy in db/db model (Janvier Labs), a mouse model of type II diabetes is assessed.

10 non diabetic and 50 diabetic mice are tested on one period. Food and water offered *ad libitum.*

In Group A (10 non-diabetic mice), glycemia levels are assessed only once. No treatment is performed on these mice.

The 50 diabetic mice are separated in 4 groups of 10-15 animals (i) subcutaneously treated with placebo or (ii) subcutaneously treated with ALF-5755 (1.25mg/kg):
Group B: 15 diabetic mice treated with placebo from D0 to D56;
Group C: 15 diabetic mice treated with ALF-5755 from D0 to D56;
Group D: 10 diabetic mice treated with placebo from D0 to D24;
Group E: 10 diabetic mice treated with ALF-5755 from D0 to D24.

During the two hours preceding the glycemia measurement performed once a week to check for diabetes level, the animals do not have access to food. Glycemia levels is obtained from vein puncture from the tail vein and classicaly assayed using glucometer (AccuCheck, Roche).

ALF-5755 or the placebo is administered daily to the animals.

Neuropathy is evaluated through tactile allodynia using Von Frey filaments and through observation of the response to mechanical stimulus of the hindpaws of the animals.

The first evaluation is performed in groups A to E before treatment (D-1), at D20 and before euthanasia at day 55 for animals from groups A to C.

Animals are placed in individual plexiglas cylinders with a mesh floor. Animals perform 30 minutes habituation sessions on days before behavorial evaluation. Animals do not stay more than one hour onto the cylinders to avoid dehydration.

The day of testing, animals are left undisturbed for a minimum of 15 minutes to acclimate to the wire grid and observation area. Tactile allodynia testing is performed using Von Frey filaments (up-down Von Frey method) applied onto the mid-plantar surface of the hindpaws.

The "up-down" Von Frey method is used to determine the mechanical force required to elicit a paw withdrawal response in 50% of animals, based on the statistical formula used to determine LD_{50S} (Dixon, 1980, Annu. Rev. Pharmacol. Toxicol. 20, 441-462; Chaplan et al., 1994, J. Neurosci. Methods 53, 55-63).

This method is well known to the man skilled in the art, as illustrated for example in J. R. Deuis, L. S. Dvorakova and I. Vetter (Methods Used to Evaluate Pain Behaviors in Rodents; Front Mol Neurosci. 2017; 10: 284), and Mills et al. (Estimating efficacy and drug ED50's using von Frey thresholds: impact of weber's law and log transformation; J Pain. 2012 Jun;13(6):519-23).

Response to mechanical stimulus is also evaluated with algometer using calibrated forceps (Rodent Pincher-analgesia meter, Bioseb) at D55 on animals from groups A, B and C.

Animals perform habituation sessions on days before behavorial evaluation.

The day of testing, animals are placed into the experiment room 2 hours before evaluation. Calibrated forceps are then applied gradually by hand at a constant rate (200g every 2 seconds) until the nociceptive response occurred. Measurement of the nociceptive threshold is repeated 3 times with intervals of approximately 5 seconds on each hindpaw.

The appearance and behavior of animals is assessed at least daily from the start and until termination of the *in vivo* phase. Any abnormal findings is recorded.

Moreover, at D24 for groups D and E, at D56 for groups B and C, all animals are euthanized and tissues are collected. Skin samples from the plantar surface of the two hindpaws from each animals (groups B to E) are collected and placed in formalin for immunohistochemistry analysis of foot intradermal nerve fiber density.

Epidermal innervation of these tissues is then evaluated.

Histology is processed by NOVAXIA (Saint Laurent Nouan, France). The plantar surface skin of the hindpaw is included in paraffin blocks. After paraffin inclusion, one slice is performed and PGP9.5 staining is done to evaluate epidermal innervation.

### SEQUENCE LISTING

**SEQ ID NO: 1** is the amino acid sequence of the HIP/PAP protein from which the N-terminal
26-amino acid signal peptide has been deleted
EEPQRELPSARIRCPKGSKAYGSHCYALFLSPKSWTDADLACQKRPSGNLVSVLSGAEGSFVSSLVKSIGNSYSY
VWIGLHDPTQGTEPNGEGWEWSSSDVMNYFAWERNPSTISSPGHCASLSRSTAFLRWKDYNCNVRLPYVCKFTD

SEQ ID NO: 2 is the amino acid sequence of the HIP/PAP protein from which the N-terminal
26-amino acid signal peptide and the 11-amino acid propeptide in the N-terminal position have
been deleted
IRCPKGSKAYGSHCYALFLSPKSWTDADLACQKRPSGNLVSVLSGAEGSFVSSLVKSIGNSYSYVWIGLHDPTQG
TEPNGEGWEWSSSDVMNYFAWERNPSTISSPGHCASLSRSTAFLRWKDYNCNVRLPYVCKFTD

SEQ ID NO: 3 is the amino acid sequence of the HIP/PAP protein from which the N-terminal
26-amino acid signal peptide has been deleted and a methionine added in N-terminal position
MEEPQRELPSARIRCPKGSKAYGSHCYALFLSPKSWTDADLACQKRPSGNLVSVLSGAEGSFVSSLVKSIGNSYS
YVWIGLHDPTQGTEPNGEGWEWSSSDVMNYFAWERNPSTISSPGHCASLSRSTAFLRWKDYNCNVRLPYVCKFTD

SEQ ID NO: 4 is the amino acid sequence of the full HIP/PAP protein
MLPPMALPSVSWMLLSCLMLLSQVQGEEPQRELPSARIRCPKGSKAYGSHCYALFLSPKSWTDADLACQKRPSGN
LVSVLSGAEGSFVSSLVKSIGNSYSYVWIGLHDPTQGTEPNGEGWEWSSSDVMNYFAWERNPSTISSPGHCASLS
RSTAFLRWKDYNCNVRLPYVCKFTD

Amino acid sequences are disclosed in the present specification that serve as references. The same sequences are also presented in a sequence listing formatted according to standard requirements for the purpose of patent matters. In case of any sequence discrepancy with the standard sequence listing, the sequences described in the present specification shall be the reference.

## Claims

1. A HIP/PAP protein, or a derivative thereof, for its use in treating and/or preventing peripheral neuropathy in an individual.

2. The HIP/PAP protein, or a derivative thereof, for use according to claim 1 in treating and/or preventing diabetic peripherical neuropathy, in particular in a diabetic or prediabetic individual, more particularly in a diabetic individual.

3. A HIP/PAP protein, or a derivative thereof, for its use in preventing a complication of peripheral neuropathy in an individual, and in particular preventing a complication of peripheral neuropathy in a diabetic or prediabetic individual, more particularly preventing a complication of diabetic peripheral neuropathy in a diabetic or prediabetic individual, in particular in a diabetic individual.

4. The HIP/PAP protein, or a derivative thereof, for use according to claim 3, wherein the complication of diabetic peripheral neuropathy is selected from the group consisting of foot deformation; diabetic foot wound, and in particular diabetic foot ulcer; foot infection; and amputations.

5. The HIP/PAP protein, or a derivative thereof, for use according to any one of claims 1 to 4, **characterized in that** the individual is a mammal, in particular a human being.

6. The HIP/PAP protein, or a derivative thereof, for use according to any one of claims 1 to 5, **characterized in that** the diabetic individual has a type II diabetes.

7. The HIP/PAP protein, or a derivative thereof, for use according to any one of claims 1 to 6, **characterized in that** the HIP/PAP protein comprises an amino acid sequence selected from the group consisting of sequences set forth as SEQ ID NO: 1, SEQ ID NO: 2, SEQ ID NO: 3 and SEQ ID NO: 4.

8. The HIP/PAP protein, or a derivative thereof, for use according to any one of claims 1 to 7, wherein said derivative comprises an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4.

9. The HIP/PAP protein, or a derivative thereof, for use according to any one of claims 1 to 8, wherein said HIP/PAP protein, or a derivative thereof, is administered to the said individual in combination with at least one agent known for being useful in the prevention and/or treatment of peripheral neuropathy, in particular selected from the group consisting of steroids, anti-inflammatory drugs, immunosuppresants and Vitamin D.

10. The HIP/PAP protein, or a derivative thereof, for use according to any one of claims 1 to 9, wherein said HIP/PAP protein, or a derivative thereof, is administered to the said individual in combination with at least one anti-diabetic agent, in particular selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.

11. The HIP/PAP protein, or a derivative thereof, for use according to any one of claims 1 to 10, **characterized in that** the HIP/PAP protein, or a derivative thereof, is in a composition comprising a physiologically acceptable medium.

12. The HIP/PAP protein, or a derivative thereof, for use according to claim 11, wherein the composition is for oral, sublingual, subcutaneous, intramuscular, intravenous, topical, local, intratracheal, intranasal or rectal administration.

13. The HIP/PAP protein, or a derivative thereof, for use according to claim 11 or 12, wherein the composition is for oral, subcutaneous, intravenous, topical or local administration.

14. The HIP/PAP protein, or a derivative thereof, for use according to any one of claims 11 to 13, wherein the composition further comprises at least one agent known for being useful in the prevention and/or treatment of peripheral neuropathy in particular selected from the group consisting of steroids, anti-inflammatory drugs, immunosuppresants and Vitamin D.

15. The HIP/PAP protein, or a derivative thereof, for use according to any one of claims 11 to 14, wherein the composition further comprises at least one anti-diabetic agent, in particular selected from the group consisting of insulin, metformin, a dipeptidyl peptidase 4 inhibitor, a glucagon-like peptide 1 receptor agonist, a sodium-glucose cotransporter 2 inhibitor and sulfonylurea and thiazolidinedione.


SEQUENCE LISTING

<110> The Healthy Aging Company

<120> HIP/PAP PROTEIN OR A DERIVATIVE THEREOF FOR TREATING PERIPHERAL
NEUROPATHY

<130> PR89778

<160> 4

<170> BiSSAP 1.3.2

<210> 1
<211> 149
<212> PRT
<213> Artificial Sequence

<223> "amino acid sequence of the HIP/PAP protein from which the N-terminal
26-amino acid signal peptide has been deleted"

<220>
<223> amino acid sequence of the HIP/PAP protein from which the
      N-terminal 26-amino acid signal peptide has been deleted

<400> 1
Glu Glu Pro Gln Arg Glu Leu Pro Ser Ala Arg Ile Arg Cys Pro Lys
1               5                   10                  15
Gly Ser Lys Ala Tyr Gly Ser His Cys Tyr Ala Leu Phe Leu Ser Pro
            20                  25                  30
Lys Ser Trp Thr Asp Ala Asp Leu Ala Cys Gln Lys Arg Pro Ser Gly
        35                  40                  45
Asn Leu Val Ser Val Leu Ser Gly Ala Glu Gly Ser Phe Val Ser Ser
    50                  55                  60
Leu Val Lys Ser Ile Gly Asn Ser Tyr Ser Tyr Val Trp Ile Gly Leu
65                  70                  75                  80
His Asp Pro Thr Gln Gly Thr Glu Pro Asn Gly Glu Gly Trp Glu Trp
                85                  90                  95
Ser Ser Ser Asp Val Met Asn Tyr Phe Ala Trp Glu Arg Asn Pro Ser
            100                 105                 110
Thr Ile Ser Ser Pro Gly His Cys Ala Ser Leu Ser Arg Ser Thr Ala
        115                 120                 125
Phe Leu Arg Trp Lys Asp Tyr Asn Cys Asn Val Arg Leu Pro Tyr Val
    130                 135                 140
Cys Lys Phe Thr Asp
145

<210> 2
<211> 138
<212> PRT
<213> Artificial Sequence

<223> "the amino acid sequence of the HIP/PAP protein from which the
N-terminal 26-amino acid signal peptide and the 11-amino acid propeptide in
the N-terminal position have been deleted"

<220>
<223> the amino acid sequence of the HIP/PAP protein from which the
      N-terminal 26-amino acid signal peptide and the 11-amino acid
      propeptide in the N-terminal position have been deleted

<400> 2
Ile Arg Cys Pro Lys Gly Ser Lys Ala Tyr Gly Ser His Cys Tyr Ala

1                   5                   10                  15
Leu Phe Leu Ser Pro Lys Ser Trp Thr Asp Ala Asp Leu Ala Cys Gln
                20                  25                  30
Lys Arg Pro Ser Gly Asn Leu Val Ser Val Leu Ser Gly Ala Glu Gly
            35                  40                  45
Ser Phe Val Ser Ser Leu Val Lys Ser Ile Gly Asn Ser Tyr Ser Tyr
    50                  55                  60
Val Trp Ile Gly Leu His Asp Pro Thr Gln Gly Thr Glu Pro Asn Gly
65                  70                  75                  80
Glu Gly Trp Glu Trp Ser Ser Ser Asp Val Met Asn Tyr Phe Ala Trp
                85                  90                  95
Glu Arg Asn Pro Ser Thr Ile Ser Ser Pro Gly His Cys Ala Ser Leu
                100                 105                 110
Ser Arg Ser Thr Ala Phe Leu Arg Trp Lys Asp Tyr Asn Cys Asn Val
        115                 120                 125
Arg Leu Pro Tyr Val Cys Lys Phe Thr Asp
    130                 135

<210> 3
<211> 150
<212> PRT
<213> Artificial Sequence

<223> "the amino acid sequence of the HIP/PAP protein from which the
N-terminal 26-amino acid signal peptide has been deleted and a methionine
added in N-terminal position"

<220>
<223> the amino acid sequence of the HIP/PAP protein from which the
      N-terminal 26-amino acid signal peptide has been deleted and a
      methionine added in N-terminal position

<400> 3
Met Glu Glu Pro Gln Arg Glu Leu Pro Ser Ala Arg Ile Arg Cys Pro
1               5                   10                  15
Lys Gly Ser Lys Ala Tyr Gly Ser His Cys Tyr Ala Leu Phe Leu Ser
                20                  25                  30
Pro Lys Ser Trp Thr Asp Ala Asp Leu Ala Cys Gln Lys Arg Pro Ser
        35                  40                  45
Gly Asn Leu Val Ser Val Leu Ser Gly Ala Glu Gly Ser Phe Val Ser
    50                  55                  60
Ser Leu Val Lys Ser Ile Gly Asn Ser Tyr Ser Tyr Val Trp Ile Gly
65                  70                  75                  80
Leu His Asp Pro Thr Gln Gly Thr Glu Pro Asn Gly Glu Gly Trp Glu
                85                  90                  95
Trp Ser Ser Ser Asp Val Met Asn Tyr Phe Ala Trp Glu Arg Asn Pro
                100                 105                 110
Ser Thr Ile Ser Ser Pro Gly His Cys Ala Ser Leu Ser Arg Ser Thr
        115                 120                 125
Ala Phe Leu Arg Trp Lys Asp Tyr Asn Cys Asn Val Arg Leu Pro Tyr
    130                 135                 140
Val Cys Lys Phe Thr Asp
145                 150

<210> 4
<211> 175
<212> PRT
<213> Artificial Sequence

<223> "the amino acid sequence of the full HIP/PAP protein"

<220>
<223> the amino acid sequence of the full HIP/PAP protein

<400> 4
Met Leu Pro Pro Met Ala Leu Pro Ser Val Ser Trp Met Leu Leu Ser
1               5                   10                  15
Cys Leu Met Leu Leu Ser Gln Val Gln Gly Glu Glu Pro Gln Arg Glu
            20                  25                  30
Leu Pro Ser Ala Arg Ile Arg Cys Pro Lys Gly Ser Lys Ala Tyr Gly
        35                  40                  45
Ser His Cys Tyr Ala Leu Phe Leu Ser Pro Lys Ser Trp Thr Asp Ala
    50                  55                  60
Asp Leu Ala Cys Gln Lys Arg Pro Ser Gly Asn Leu Val Ser Val Leu
65                  70                  75                  80
Ser Gly Ala Glu Gly Ser Phe Val Ser Ser Leu Val Lys Ser Ile Gly
                85                  90                  95
Asn Ser Tyr Ser Tyr Val Trp Ile Gly Leu His Asp Pro Thr Gln Gly
            100                 105                 110
Thr Glu Pro Asn Gly Glu Gly Trp Glu Trp Ser Ser Ser Asp Val Met
        115                 120                 125
Asn Tyr Phe Ala Trp Glu Arg Asn Pro Ser Thr Ile Ser Ser Pro Gly
    130                 135                 140
His Cys Ala Ser Leu Ser Arg Ser Thr Ala Phe Leu Arg Trp Lys Asp
145                 150                 155                 160
Tyr Asn Cys Asn Val Arg Leu Pro Tyr Val Cys Lys Phe Thr Asp
                165                 170                 175